(19) Europäisches Patentamt
European Patent Office
Office européen des brevets

(11) EP 1 633 342 B1

(12) **EUROPEAN PATENT SPECIFICATION**

(45) Date of publication and mention of the grant of the patent:
**07.04.2010 Bulletin 2010/14**

(21) Application number: **04734795.0**

(22) Date of filing: **26.05.2004**

(51) Int Cl.:
***A61K 31/40*** *(2006.01)* ***A61K 31/19*** *(2006.01)*
***A61K 31/407*** *(2006.01)*

(86) International application number:
**PCT/BR2004/000077**

(87) International publication number:
**WO 2004/105678 (09.12.2004 Gazette 2004/50)**

# (54) PHARMACEUTICAL COMPOSITION BASED ON KETOROLAC OR ONE OF ITS SALTS PHARMACEUTICALLY ACCEPTABLE AND USE OF KETOROLAC OR ITS SALTS IN PHARMACEUTICAL COMPOSITIONS

PHARMAZEUTISCHE ZUSAMMENSETZUNG AUF BASIS VON KETOROLAC ODER EINEM SEINER PHARMAZEUTISCH ANNEHMBAREN SALZE UND VERWENDUNG VON KETOROLAC ODER SEINEN SALZEN IN PHARMAZEUTISCHEN ZUSAMMENSETZUNGEN

COMPOSITION PHARMACEUTIQUE A BASE DE KETOROLAC OU UN DE SES SELS ACCEPTABLE AU PLAN PHARMACEUTIQUE, UTILISATION DU KEROTOLAC OU UN DE SES SELS DANS DES COMPOSITIONS PHARMACEUTIQUES

(84) Designated Contracting States:
**AT BE BG CH CY CZ DE DK EE ES FI FR GB GR HU IE IT LI LU MC NL PL PT RO SE SI SK TR**

(30) Priority: **02.06.2003 BR 0302736**

(43) Date of publication of application:
**15.03.2006 Bulletin 2006/11**

(73) Proprietor: **Nature's Plus Farmacêutica Ltda.**
**13186-481 Hortolândia (BR)**

(72) Inventors:
- **VANDONI, Guido**
  **I-20050 Correzzana (IT)**
- **OLIANI, Carlo**
  **13186-481 Hortolândia (BR)**
- **COELHO, Adriano**
  **13186-481 Hortolândia (BR)**
- **ZANIBONI, Heny**
  **20151 Milani (IT)**

(74) Representative: **Alves Moreira, Pedro**
**Rua do Patrocinio, 94**
**1399-019 Lisbon (PT)**

(56) References cited:
**US-A- 5 288 497** **US-A- 5 626 838**
**US-B2- 6 685 951**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

The present invention refers to the use of ketorolac or one of its salts, acceptable from pharmaceutical point of view, for preparation of a pharmaceutical composition in tablets, for sublingual administration, to accelerate the pharmaceutical response, especially the analgesic response by the use of ketorolac.

### State of the Art

Ketorolac is a medicament inhibitor of prostaglandin synthesis, which formula is

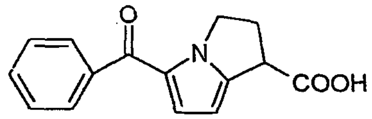

notably known by its anti-inflammatory, analgesic and antipyretic action as described in Patent US 4,089,969, in which tromethamol salt is used in its racemic form.
Pharmaceutical formulations with analgesic and antipyretic action, containing R form, were also described in the state of the art, as can be noted in the patent document EP 674511. It should also be emphasized that ketorolac may be used for the therapy in periodontal affection treatments, as described in patent document WO 91/13609, and for the treatment of carcinomas of the scaly cells in the oral cavity or of the oropharynx, as taught in the patent document WO 96/28144.
In the several indications mentioned in the literature, ketorolac was proposed using several administration routes. For example, Patent US 4,089,969 suggests the oral, parenteral or topic administration, in several pharmaceutical forms, particularly in tablets, suppository, pills, capsules, powder, solutions, suspensions, emulsions, creams, lotions and unguents.
However, patent document WO 91/13609 suggests the application of ketorolac through dentifrices, solutions for mouthwash, spray for oral cavity or dental solutions. The latter type of ketorolac application is also suggested in patent document WO 96/28144.
The Patent US 6,090,368 describes the nasal spray, whereas patent document WO 99/09954 describes a compound for skin topic administration and patent document EP 668759 describes ketorolac administration by transdermic via.
The Patent US 5,288,497 (STANLEY et al.) refers to compositions and methods of manufacture for producing medicament compositions for use in administering potent, fast-acting drugs transmucosally, that is, through the mucosal tissue of the mouth, pharynx and esophagus, thereby avoiding the problems of both injection and oral administration. Such compositions and methods are useful in administering drugs in a dose-to-effect manner such that sufficient drug is administered to produce precisely the desired effect. The invention relates to a manufacturing technique that enables both lipophilic and nonlipophilic therapeutic agents to be incorporated into a flavored dissolvable matrix material and to attach the matrix mixture onto an appliance or holder. The present Invention is not based on matrix material, but on essential excipients, composed by ternary mixtures lactose/sorbitol/cellulose that will transport one medicine which will be absorbed by buccal mucosa, whereby the scope of the present invention is not the same as the Patent US 5,288,497.
In some embodiments of said Patent US 5,288,497, specific confectionery components are combined in order for the mixture to form an integral solid mass. These components may include, for example, compressible confectioner's sugar, sorbitol, mannitol, and maltodextrin. But this mixture (sorbitol, mannitol, and maltodextrin) is only used as sweetening agents. In the present Invention the excipients from ternary mixture are, only and exactly, lactose/sorbitol/cellulose and are used as diluents in such specific proportions that enable them to pass through the mucosa at desired velocity. Again, the scope is not the same.
In yet other embodiments of Patent US 5,288,497, therapeutic agents may be combined with hydrogels or gelatins to form a dissolvable drug delivery system. The Present Application does not use this mechanism for drug delivery.
The Patent US 5,288,497 generally requires high temperatures to form a molten candy matrix and which can cause degradation of some drugs. This problem is avoided by the present invention, since it does not use this mechanism for drug delivery.
Referring now to Patent US 5,626,838 (CAVANAUGH, JR), it relates to methods and compositions for prevention or treatment of primary and/or reccuring squamous cell carcinomas of the oral cavity or oropharynx comprising topical administration, to the oral cavity or oropharynx, of a composition providing from about 0.001% to about 0.2% by weight,

of ketorolac to the oral cavity or oropharynx, alone or as an adjunct to surgery and/or radiation therapy.

One aspect of the Patent US 5,626,838 is compositions comprising a safe and effective amount, preferably from about 0.001% to about 5%, and a pharmaceutically acceptable topical, oral carrier. Topical, oral carrier, denotes a carrier for the non-steroidal anti-inflammatory drugs (NSAID), selected from the group consisting of aspirin, ibuprofen, naproxen, indomethacin, piroxicam, flurbiprofen, meclofenamate sodium, ketoprofen, tenidap, tebufelone, ketorolac, and mixtures thereof, which results in a composition which is administered topically to the oral cavity, held therein for a period of from about 15 seconds to about 10 minutes, and them is largely expectorated rather than being swallowed. Such compositions are in the form of a toothpastes mouthwashes, mouthsprays, dental solutions, and like, and comprise about 0.001% to about 0.2%, by weight of ketorolac and is free of hyaluronic acid. So, in view of the aforementioned the formulations are very distinct, either qualitative or quantitatively and the scope is not the same.

With respect to Patent US 6,685,951 (CUTLER), it relates to a method of treating migraine comprising: the application of a spray or an aerosol to the sublingual mucosa of a patient wherein said spray or aerosol consists of one active ingredient and one or more inactive ingredients, wherein said active ingredient is dihydroergotamine (DHE) and wherein at least one of said one or more inactive ingredients is selected from the group consisting of cyclodextrins, dextrans, sugar alcohols and mixtures thereof.

In one embodiment of the Patent US 6,685,951, it is contemplated that DHE is combined with inactive ingredients. It is also contemplated that other active ingredients, like analgesics or anesthetics in addition to DHE, may be added to the pharmaceutical preparation.

In a particular embodiment, non-steroidal anti-inflammatory drugs (NSAID) are contemplated, in a preferred embodiment ketorolac tromethamine or diclofenac agent is administered. These other active ingredients are administered in combination with the sublingual dose of DHE. Such co-administration may be sublingual, oral, rectal, buccal, injectable, nasal, etc. But, the Patent US 6,685,951 invention does not mention if the administration of non-steroidal anti-inflammatory drugs is in the same dosage form as the DHE. The invention define that these drugs are administered either simultaneously or sequentially with DHE. The example does not describe non-steroidal anti-inflammatory drugs in the same formulation.

In another embodiment, it is contemplated by Patent US 6,685,951 that the carrier used may comprise sugar, mannitol and lactose, and it is also mentioned that a variety of polymers can be used as bio/mucoadhesion promoting agents, including cellulose derivatives. However, all of these compounds are known to the persons skilled in the art and so they could be used in several applications.

The Patent US 6,685,951 does not disclose neither suggests the teachings of the present invention. It is never expected that a person skilled in the art can find the present invention based on said patent.

Currently, ketorolac, in the form of tromethanol salt is used by oral via in tablets of 10 mg or dropwise, in a solution of 2%, by injectable via, being provided in flasks of 10 or 30 mg, and by rectal via in suppositories of 30 mg.

Consequently, in order to obtain a rapid anti-inflammatory, antipyretic and analgesic action of ketorolac, the single current possibility is to make use of the injectable administration via, which knowingly causes slight illness and discomfort to the patient. Particularly due to its use as analgesic, a greater speed of ketorolac action assumes a significant importance.

## Summary of the Invention

It was recently proved that with ketorolac administration or one of its salts acceptable from pharmaceutical viewpoint, more particularly its tromethamol salt by sublingual via, the absorption of the active principle is more rapidly obtained than in comparison with the oral administration, thus resulting in a more rapid therapeutic action with plasmatic levels of the active principle equal or very close to those obtained after the traditional oral administration, which results in a therapeutic activity very close, if not equal to the previous once, or even better.

On the other hand, it was proved that a pharmaceutical (composition composed by the essential carrier containing 10-15% in weight of ketorolac or one of its salts acceptable from pharmaceutical point of view, preferably tromethamol ketorolac, and with at least 60% of the total weight of the compound represented by *essential excipients,* composed by ternary mixture of lactose/sorbitol/cellulose in the respective weight percentages (in relation to the total weight of the compound) of 45-50%/5-9%/10-17%, is rapidly absorbed by sublingual via, allowing to achieve the hematic levels of ketorolac rapidly, with sufficient value to assure the therapeutic activity desired in the above mentioned indications, particularly in relation to the analgesic property.

The term "essential carrier" refers to the pharmaceutical composition containing a mixture of the active principle of ketorolac compound or one of its salts acceptable from pharmaceutical viewpoint, particularly its tromethamol salt, an excipient necessary to compose the formulation, which is presented in the form of sublingual tablets for oral administration, specially including the essential excipients. The term "essential excipients" refers to the mixture of the excipients or the ternary mixture lactose/sorbitol/cellulose in specific proportions (lactose (45-50% in weight)/sorbitol (5-9% in weight)/cellulose (10-17% in weight). The term "ternary mixture" refers to the three excipients, lactose/sorbitol/cellulose. Hereinafter, the terms essential carrier, essential excipients and ternary mixture will appear in the present document

meaning the foregoing explanation.

The term "ketorolac" represents the International Common Denomination" (DCI) of the racemic form of 5-benzyl-2,3-dihydro-1H-pyrrolizin-1-carboxylic acid.

The term tromethamol" represents the DCI of 2-amino-2-(hydroxymethyl)-1,3-propanediol, also indicated as "tromethamine" in USP dictionary of pharmaceutical products.

The expression "tromethamol ketorolac", specifically designates the tromethamol ketorolac salt.

**Detailed Description of the Invention**

According to one of its aspects, the present invention refers to the use of ketorolac or one of its salts acceptable from pharmaceutical viewpoint, for the preparation of a pharmaceutical composition for sublingual administration destined to accelerate the pharmacological response to ketorolac without making use of the injectable via. It is understood by the term "sublingual", the application of the active principle through the administration of the pharmaceutical composition in the said sublingual form, i.e., under the tongue or gingiva, i.e., placing the tablet between the cheek and the gingiva.

It is understood that the expression "pharmacological response to ketorolac" refers to the response due to the systemic action of ketorolac itself or one of its salts acceptable from pharmaceutical viewpoint, preferably the tromethamol salt, in the treatment of the aforementioned diseases.

One of the advantageous uses of ketorolac or one of its salts acceptable from pharmaceutical viewpoint, consists of the preparation of a pharmaceutical composition for sublingual administration with the purpose of accelerating the analgesic response of ketorolac without making use of the injectable via. Even more advantageous, the invention refers to the use of tromethamol ketorolac for preparation of the pharmaceutical compounds for sublingual administration destined to accelerate the analgesic response of ketorolac without making use of the injectable via.

According to one of its other aspects, the present invention provides a pharmaceutical composition for sublingual use, due to the quality of one of its principle actives, reaching from 10 to 15% in weight of ketorolac or one of its salts acceptable from pharmaceutical viewpoint, mixed with a pharmaceutical excipient formed by, at least, 60% in relation to the total weight of the excipients of a diluent composed of a polyalcohol derivative from reduction of a monosaccharide and a carbohydrate, at least.

In the present context, unless otherwise indicated, the specific indication of the percentages is given in weight and refers to the total weight of the composition.

The referred pharmaceutical composition may promote the acceleration of ketorolac pharmacological response. It is composed of tablets for sublingual administration containing from 2 to 15 mg of ketorolac or one of its salts acceptable from pharmaceutical viewpoint. The same is particularly destined to the rapid induction of the analgesia in patients requiring this rapid induction.

The recommended dosage unit of the referred to agonists is composed of 2.5; 5 or 10 mg of ketorolac, preferably tromethamol ketorolac mixed with excipients, of which, at least, 60% is composed of a polyalcohol derivative of a monosaccharide and of, at least, a carbohydrate, and eventually, other excipients as lubricants, aggregate, sweetening, taste correctors (flavors) and casually, disaggregating agents used in the manufacture of pills and tablets for sublingual use.

Are considered as advantageous excipients or essential excipients, those excipients used in quantity of at least 60% in weight in relation to the total weight of the composition, the carbohydrates as cellulose powder, microcrystalline cellulose or sodium carboxymethylcellulose, lactose, corn or potato starch, natural or modified, or mixtures thereof and polyalcohol derivatives from reduction of monosaccharides as D-mannitol, L-glucitol and especially, D-glucitol or sorbitol, which may also act as sweetening agent.

In relation to the other excipients, the lubricants as polyethyleneglycol, especially polyethyleneglycol 6000 are used in quantities corresponding to 0-5% by weight, the aggregative as microcrystalline silica are advantageously being used with weight ranging between 0,5 and 5%, the disaggregating agent as polyvinyl pyrrolidone generally represents from 5 to 10% by weight. In practice, carbohydrates and derivatives thereof, essentially compose the excipients of the composition of the present invention.

Due to the sublingual use of the pharmaceutical preparation of the present invention, the sweetening and/or the flavors constitute an essential presence and will be chosen by experts of the area, in relation to the organoleptic characteristics of any of the active principles. The natural sweetening are advantageously being used, including mannitol and sorbitol, which in this case are part of the diluents, the latter being used in quantities corresponding to 3-9%, whereas the synthetic sweetening as sodium saccharine or aspartame are used in quantities of 0.1-5%.

The flavors incorporated to the composition may also be chosen among the flavors of synthetic or natural oils, encompassing plant, leaf, flower, fruit extracts and combinations thereof, as cinnamon, *mentha piperita*, anise, cedar leaves, bitter almond, citrus fruits, especially orange and lemon, chamomile and grapefruit. The tropical flavors as vanilla or eucalyptus flavor, and fruit essences, especially apple, pear, peach, raspberry, cherry and grape may be advantageously used.

Generally, the flavors are present in quantities ranging from 0.05% to 4% of the total weight of the compound. The preferred flavors are the tropical flavors and those that give mint or fruit taste, particularly of grape, cherry or citric fruits, especially orange, lemon or mixtures thereof.

According to another aspects thereof, the present invention provides a pharmaceutical composition that comprises 10-15% by weight of ketorolac or one of its salts acceptable from pharmaceutical viewpoint, preferably from 60 to 75% of the total weight of the compound of an essential excipient, composed of a ternary mixture of lactose/sorbitol/cellulose, in the respective weight percentages (in relation to the total weight of the compound) of 45-50% /5-9% /10-17%, preferably of 45-50% /5-8%/12-14%. Preferably, in the referred to essential excipient, the relation lactose/cellulose is of approximately 2.5/1 close to 3.5/1, and sorbitol represents 6-8% of the total weight of the compound. The referred to pharmaceutical composition is found in dosage units of, for example, tablets containing from 2 to 15 mg, preferably 2.5; 5 or 10 mg of ketorolac or one of its salts acceptable from pharmaceutical viewpoint, preferably tromethamol ketorolac. This type of tablets is especially appropriate for sublingual administration.

According to a preferential aspect of the invention, the referred to essential excipient is composed of a mixture of lactose (45-48% by weight)/sorbitol (5-8% by weight)/cellulose (12-15% by weight), which weight relation lactose/sorbitol/cellulose is of $3\pm0,5/\ 0,6\pm0,2/1$, eventually in a mixture with other excipients acceptable from pharmaceutical viewpoint.

The essential excipients, composed of the mixture lactose/sorbitol/cellulose in the referred to weight percentages, in relation to the total weight of the compound is preferably a mixture with a proportion of approximately 3/0.4-0.7/1, containing ternary mixture forming the essential excipients in the weight percentages, each one always referring to the total weight of the compound, lactose (45-50%)/sorbitol (5-9%)/cellulose (10-17%).

The referred to composition is particularly indicated for sublingual administration.

In addition to the essential excipients, lactose/sorbitol/cellulose, the composition of the invention may contain other excipients, especially disaggregating agents, as polyvinyl pyrrolidone, corn or potato starch, eventually modified, lubricants as magnesium stearate, aggregative agents, as methylcellulose, sodium carboxymethylcellulose, polyethyleneglycol, silica microcrystalline, colloidal silicon dioxide, magnesium and aluminum silicate, dyers as titanium dioxide, sweetening and flavors as previously mentioned.

The composition of the present invention may contain excipient that give it effervescence during the permanence under the tongue. The advantageous excipients of this type of composition are the mixtures of acids and carbonates, adequately prepared in accordance with the usual techniques, especially citric or tartaric acid and sodium, potassium or magnesium carbonate or bicarbonate.

The tablets for sublingual administration of the present invention may be manufactured in accordance with the classic methods used in pharmaceutical technique, for example, by direct compression, by humid granulation or using technology of active principle incorporation in micro-granules, microsphere or micro-emulsions, allowing better absorption of the referred to active principle.

The composition of the present invention will be prepared according to methods of pharmaceutical technique. In practice, the calculated quantity of tromethamol ketorolac, of sorbitol, preferably micro-granulated and one aggregative, for example, sodium carboxymethyl-cellulose, will pass through a sieve and mixed for 10-15 minutes. To this mixture will be added the calculated quantity of lactose, cellulose, preferably micronized, of a disaggregate as polyvinyl pyrrolidone or crospovidone, for example, Polyplasdone XL, of a ligand as colloidal silicon, for example, Syloid 244, of a flavor and a sweetening, making all these excipients pass through a sieve of 30 mesh and mixing everything for further 10-15 minutes. To the mixture thus obtained will be added the calculated quantity of a lubricant, for example, magnesium stearate or polyethyleneglycol, mixing for some minutes and making the compression of the mixture with appropriate punctures.

The composition of the present invention, after administration of a dose of 10 mg, enables the hematic rates of ketorolac to be rapidly reached, thus ensuring rapid analgesic or anti-inflammatory action.

The bioavailability after the sublingual administration of a single dose of 10 mg of tromethamol ketorolac was compared with the same single dose of the active principle in conventional oral tablets, in a study with twelve healthy male volunteers recruited after the respective information on the nature and characteristics of the active principles and also on the scope of the study. The referred to study, composed of four treatment periods, was carried out according to a crossed and randomized scheme.

All individuals received the pre-established dose of the active principle in the form of conventional oral tablets, in the form of sublingual tablets, by endovenous and intramuscular via, within a washout period of at least seven days after the first treatment and each subsequent treatment, according to a pre-established list of randomization. The sublingual tablets were placed under the tongue in correspondence with the venous plexus, until its complete dissolution, whereas the conventional oral tablets were swallowed unbroken with 150 ml of mineral water. Five ml of venous blood were collected in the periods 0-5-10-20-40 minutes, 1-2-4-8 and 24 hours after the treatment. Table I shows the average plasmatic rates measured after the single doses mentioned.

Table I

| | 0 | 5 minutes | 10 minutes | 20 minutes | 40 minutes | 1 hour | 2 hours | 4 hours | 8 hours | 24 hours |
|---|---|---|---|---|---|---|---|---|---|---|
| **E.V.** | 0 | 315,83 ± 136,09 | 2858,89 ± 680,35 | 1096,58 ± 376,46 | 794,31 ± 274,46 | 637,20 ± 243,46 | 504,58 228,98 | 332,41 ±177,30 | 156,80 ± 83,02 | 16,21 ± 18,04 |
| **I.M.** | 0 | 5,70 ± 10,39 | 119,09 ± 56,83 | 245,20 ± 115,17 | 543,08 ± 316,31 | 720,04 ± 201,80 | 448,85 ± 117,88 | 323,04 ± 103,29 | 193,88 ± 63,74 | 25,63 ± 19,82 |
| **Conventional Tablet** | 0 | 0 | 43,73 ± 42,88 | 227,15 ± 73,17 | 660,69 ± 303,43 | 730,60 ± 235,35 | 480,28 ± 232,68 | 317,03 ± 190,96 | 161,04 ± 87,83 | 26,29 ± 22,94 |
| **Sublingual Tablet** | 0 | 43,28 ± 31,81 | 205,26 ± 95,16 | 736,30 ± 275,13 | 776,28 ± 287,80 | 626,88 ± 260,98 | 429,04 ± 212,33 | 305,71 ± 182,59 | 158,35 ± 97,70 | 25,41 ± 25,46 |

In above table, exactly in the first minutes after the sublingual administration, can be observed a tendency for obtaining hematic rates higher in relation to those obtained after administration of the same dose by oral or intramuscular via, whereas the other plasmatic rates can be easily overcome.

Table II shows the data referring to the several pharmacokinetic parameters (average ± standard deviation) referring to sublingual, conventional oral, endovenous and intramuscular administration of tromethanol ketorolac. Especially the $AUC_{0-t}$ informed in the Table, i.e., the area under the time curve 0 up to time T (in this case, at hour 24), its logarithm $AUC_{0-inf}$ (in ng/ml/h) i.e., the area under the curve according to the formula

$$AUC_{0-inf} = AUC_{0-t} + C_{t/b}$$

in which $C_t$ is the plasmatic concentration (in ng/ml) estimated at time T (in this case, at hour 24) and b is the slope of the curve of the washout phase, being its logarithm, the $C_{max}$ (in ng/ml), i.e., the peak of maximum concentration, its logarithm and the $T_{max}$, i.e., the time (in hours) in which the maximum plasmatic concentration peak is compared.

Table II

| PHARMACOKINETIC PARAMETERS | Sublingual Tablets | Conventional Tablets | E.V. | I.M. |
|---|---|---|---|---|
| AUC $_{0-t}$ (ng/ml/h) | 237972,81 ± 141094,58 | 233989,13 ± 118781,73 | 260432,85 ± 41307,54 | 260002,03 ± 96077,99 |
| AUC $_{0-inf}$ (ng/ml/h) | 258205,10 ± 143740,06 | 263791,13 ± 124504,49 | 281323, 89 ± 140122,03 | 278349, 95 ± 92737,20 |
| Log AUC $_{0-t}$ | 5,30 ± 0,28 | 5,31 ± 0,24 | 5,36 ± 0,22 | 5,38 ± 0,22 |
| Log AUC $_{0-inf}$ | 5,34 ± 0,27 | 5,37 ± 0,24 | 5,40 ± 0,21 | 5,41 ± 0,19 |
| $C_{max}$ (ng/ml) | 991,47 ± 219,40 | 871,28 ± 236,90 | 2858,89 ± 680,35 | 835,31 ± 196,13 |
| Log $C_{max}$ | 2,99 ± 0,10 | 2,92 ± 0,13 | 3,45 ± 0,10 | 2,91 ± 0,11 |
| $T_{max}$ (min) | 33,33 ± 13,03 | 53,33 ± 9,85 | 10 - | 51,67 ± 10,30 |

From the statistic analyses made from all pharmacokinetic parameters, using the analysis of variance (ANOVA) and the test of Bonferroni, and also only on the $T_{max}$ parameter, the tests for the data in pairs of Wilcoxon, did not result in significant differences in the treatments with reference to $logAUC_{0-inf}$, either in ANOVA test or in Bonferroni test, while a significant difference can be observed between the endovenous treatment and the other ways of administration with reference to $logC_{Max}$. With reference to $T_{max}$ parameter, it was observed that either in ANOVA test or in Bonferroni and Wilcoxon tests, there is a significant difference between the treatment by sublingual via and those by conventional oral or intramuscular via. It was especially evidenced that the sublingual treatment presents a $T_{max}$ significantly shorter in relation to the conventional oral or intramuscular treatment, considered equal between it.

The following examples explain the invention, however, without limiting the same.

## EXAMPLE 1

A mixture of 1.000 kg of tromethamol ketorolac, 0.500 kg of micro-granulated sorbitol and 0.200 kg of sodium carboxymethylcellulose is passed through a sieve of 30 mesh, and after that, mixed for 10 minutes. Add to this mixture, 3.375 kg of lactose, 1.125 kg of micronized cellulose, 0.150 kg of sodium saccharine, 0.6 kg of Polyplasdone XL, 0.100 kg of Syloid 244 and 0.250 kg of lemon flavor, previously passed through a sieve of 30 mesh. Slurry the mixture for 10 minutes. Add 0.200 kg of magnesium stearate previously passed through a sieve of 30 mesh. Slurry the end-mixture for 3 minutes. Promote the compression in an alternative or rotating compression machine, provided with punctures with 6 mm diameter. Thus, 100,000 tablets will be obtained with a weight of 75 mg each, with a formulation showing the following composition:

| | |
|---|---|
| Tromethamol Ketorolac | 10.00 mg |
| Lactose | 33.75 mg |
| Sorbitol | 5.00 mg |

(continued)

| | |
|---|---|
| Micronized Cellulose | 11.25 mg |
| Sodium Carboxymethylcellulose | 2.00 mg |
| Sodium Saccharine | 1.50 mg |
| Polyplasdone XL | 6.00 mg |
| Syloid 244 | 1.00 mg |
| Lemon flavor | 2.50 mg |
| Magnesium stearate | 2.00 mg |

The tromethamol ketorolac tablets for sublingual administration thus obtained, contain 13.33% of active principle and 66.67% of essential excipients, among which lactose represents 45% of the total compound, sorbitol representing 6.67% of the total compound and cellulose representing 15% of the total compound, being the proportion of lactose/sorbitol/cellulose of 3/0.44/1.

**EXAMPLE 2**

Carrying out the same operation described in Example 1 above, with 1.000 kg of tromethamol ketorolac, 4.500 kg of Cellactose, composed of a mixture containing 75% of lactose and 15% of cellulose, 0.500 kg of micro-granulated sorbitol, 0.200 kg of sodium carboxymethylcellulose, 0.600 kg of polyvinyl pyrrolidone (Collidon C1 BASF), 0.250 kg of lemon flavor (15203-71/MD-Givaudan), 0.100 kg of microcrystalline silica (Syloid 244), 0.200 kg of magnesium stearate and 0.150 kg of sodium saccharine 100,000 tablets will be prepared with a weight of 75 mg each, presenting the following composition:

| | |
|---|---|
| Tromethamol Ketorolac | 10.00 mg |
| Lactose | 33.75 mg |
| Microcrystalline Cellulose | 11.25 mg |
| Microgranulated Sorbitol | 5.00 mg |
| Polyvinyl pyrrolidone | 6.00 mg |
| Sodium Carboxymethylcellulose | 2.00 mg |
| Lemon flavor | 2.50 mg |
| Sodium Saccharine | 1.50 mg |
| Magnesium stearate | 2.00 mg |
| Microcrystalline Silica | 1.00 mg |

The tromethamol ketorolac tablets for sublingual administration thus obtained contain 13.33% of active principle and 66.67% of essential excipients, among which lactose represents 45% of the total compound, sorbitol representing 6.67% of the total compound and cellulose representing 15% of the total compound, being 3/0.44/1 the respective proportion of lactose/sorbitol/cellulose.

## Claims

**1.** A pharmaceutical composition for sublingual administration comprising:

i) 10-15% by weight of ketorolac or a salt thereof,
ii) at least 60% by weight of a ternary mixture which is composed by:

40-50% by weight of lactose,
5-8% by weight of sorbitol,
12-14% by weight of cellulose,

iii) other excipients.

**2.** A pharmaceutical composition according to claim 1, wherein said other excipients are lubricants, dyers, taste correctors, aggregating and disaggregating agents, effervescence agents and the like.

3. A pharmaceutical composition according to claim 1, comprising 2 to 15 mg of ketorolac or a salt thereof.

4. A pharmaceutical composition according to claims 1 to 7, wherein the salt of ketorolac is tromethamol ketorolac.

5. Use of pharmaceutical composition according to preceding claims, **characterized by** being intended to accelerate the pharmacological responses to ketorolac without making use of an injectable route.

**Patentansprüche**

1. Pharmazeutische Zusammensetzung für die sublinguale Verabreichung, umfassend:

   i) 10-15 Gew.-% Ketorolac oder eines Salzes davon,
   ii) mindestens 60 Gew.-% eines ternären Gemischs, bestehend aus:

      40-50 Gew.-% Lactose,
      5-8 Gew.-% Sorbit,
      12-14 Gew.-% Cellulose,

   iii) andere Excipienten.

2. Pharmazeutische Zusammensetzung nach Anspruch 1, wobei es sich bei den anderen Excipienten um Gleitmittel, Farbstoffe, Geschmackskorrektoren, Aggregations- und Auflösemittel, Aufschäummittel und dergleichen handelt.

3. Pharmazeutische Zusammensetzung nach Anspruch 1, die 2 bis 15 mg Ketorolac oder eines Salzes davon umfasst.

4. Pharmazeutische Zusammensetzung nach Anspruch 1 bis 7, wobei es sich bei dem Salz von Ketorolac um Ketorolac-Trometamol handelt.

5. Verwendung einer pharmazeutischen Zusammensetzung nach den vorhergehenden Ansprüchen, **dadurch gekennzeichnet, dass** sie dafür bestimmt ist, die pharmakologischen Reaktionen auf Ketorolac zu beschleunigen, ohne dass ein Injektionsweg verwendet wird.

**Revendications**

1. Composition pharmaceutique destinée à l'administration sublinguale, comprenant :

   i) 10-15 % en poids de kétorolac ou d'un sel de celui-ci,
   ii) au moins 60 % en poids d'un mélange ternaire qui est composé de :

      40-50 % en poids de lactose,
      5-8 % en poids de sorbitol,
      12-14 % en poids de cellulose,

   iii) d'autres excipients.

2. Composition pharmaceutique selon la revendication 1, **caractérisée en ce que** lesdits autres excipients sont les lubrifiants, les colorants, les correcteurs de goût, les agents agrégants et désagrégeants, les agents d'effervescence et similaires.

3. Composition pharmaceutique selon la revendication 1, comprenant de 2 à 15 mg de kétorolac ou d'un sel de celui-ci.

4. Composition pharmaceutique selon les revendications 1 à 7, **caractérisée en ce que** le sel de kétorolac est le kétorolac trométamol.

5. Utilisation d'une composition pharmaceutique selon les revendications précédentes, **caractérisée en ce qu'**elle est destinée à accélérer les réponses pharmacologiques au kétorolac sans faire appel à une voie injectable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4089969 A
- EP 674511 A
- WO 9113609 A
- WO 9628144 A
- US 6090368 A
- WO 9909954 A
- EP 668759 A
- US 5288497 A, STANLEY
- US 5626838 A, CAVANAUGH, JR
- US 6685951 B, CUTLER